# EUROPEAN PATENT APPLICATION

(11) **EP 4 592 020 A1**
(43) Date of publication of application: **30.07.2025**
(21) Application number: 24221790.9
(22) Date of filing: 19.12.2024
(51) Int. Cl.: B23K 31/12

(54) **OPTICAL DEVICE AND METHOD FOR TRAINING MACHINE LEARNING MODEL FOR WELDING DEFECT INSPECTION**

(30) Priority: 12.01.2024 KR 20240005312
(71) Applicant: SAMSUNG SDI CO., LTD., Yongin-si, Gyeonggi-do 17084 (KR)
(72) Inventor: Kim, Joonkil, 17084 Yongin-Si, Gyeonggi-do (KR); Oh, Taegyeong, 17084 Yongin-Si, Gyeonggi-do (KR); Yu, Juheon, 17084 Yongin-Si, Gyeonggi-do (KR); Lee, Haeyun, 17084 Yongin-Si, Gyeonggi-do (KR); Kim, Donghyun, 17084 Yongin-Si, Gyeonggi-do (KR); Lee, Jonghoon, 17084 Yongin-Si, Gyeonggi-do (KR); Kim, Soohong, 17084 Yongin-Si, Gyeonggi-do (KR); Park, Jihyang, 17084 Yongin-Si, Gyeonggi-do (KR); Lee, Sangwoo, 17084 Yongin-Si, Gyeonggi-do (KR); Lagunovsky, Dmitry, 17084 Yongin-Si, Gyeonggi-do (KR); Hwang, Sunuk, 17084 Yongin-Si, Gyeonggi-do (KR)
(74) Representative: Gulde & Partner

(57) **Abstract**

An optical device may include an infrared detection unit that detects heat generated at a weldment during welding, a visible light detection unit that detects plasma generated at the weldment and a spectroscopic unit that detects wavelength-specific luminosity at the weldment, wherein the optical device determines an occurrence of welding defects at the weldment based on the detected heat, the detected plasma, and/or the detected wavelength-specific luminosity of the weldment.

## Description

### FIELD

The present disclosure relates to an optical device and a method for training a machine learning model for welding defect inspection.

### BACKGROUND

Unlike primary batteries that are not designed to be (re)charged, secondary (or rechargeable) batteries are batteries that are designed to be discharged and recharged. Low-capacity secondary batteries are used in portable, small electronic devices, such as smart phones, feature phones, notebook computers, digital cameras, and camcorders, while large-capacity secondary batteries are widely used as power sources for driving motors in hybrid vehicles and electric vehicles and for storing power (e.g., home and/or utility scale power storage). A secondary battery generally includes an electrode assembly composed of a positive electrode and a negative electrode, a case accommodating the same, and electrode terminals connected to the electrode assembly.

Laser welding is widely employed in a production process of secondary batteries, and various inspection methods are used to measure the quality of a weldment made through the laser welding.

The above information disclosed in this Background section is for enhancement of understanding of the background of the present disclosure, and therefore, it may contain information that does not constitute related (or prior) art.

### SUMMARY

The present disclosure relates to an optical device and a method for training a machine learning model, a non-transitory computer-readable storage medium storing instructions, and an apparatus (e.g., system) for performing welding defect inspection in less (e.g., minimized) time without destroying an inspection target object.

However, the technical problem to be solved by the present disclosure is not limited to the above problem, and other problems not mentioned herein, and aspects and features of the present disclosure that would address such problems, will be clearly understood by those skilled in the art from the description of the present disclosure below.

To solve the above technical problem, a system according to an embodiment of the present disclosure may include an optical device which may include an infrared detection unit that detects heat generated at a weldment during welding, a visible light detection unit that detects plasma generated at the weldment and a spectroscopic unit that detects wavelength-specific luminosity at the weldment, wherein the system and/or the optical device may determine an occurrence of welding defects at the weldment based on the detected heat, the detected plasma, and/or the detected wavelength-specific luminosity of the weldment.

According to one or more embodiments, welding light generated during welding of the weldment may pass through the infrared detection unit, the visible light detection unit, and the spectroscopic unit in sequence.

According to one or more embodiments, the infrared detection unit may include a first optical filter that reflects or transmits a portion of welding light generated during welding of the weldment and an infrared sensor that receives a first portion of the welding light reflected from the first optical filter, wherein the visible light detection unit may receive a second portion of the welding light that has transmitted through the first optical filter, a wavelength band of the first portion may be included in an infrared band, and a wavelength band of the second portion may be included in a visible light band.

According to one or more embodiments, the wavelength band of the first portion may be determined based on a type of welding target.

According to one or more embodiments, the visible light detection unit may include a second optical filter that splits received light, and a visible light sensor that receives a third portion of the welding light, the third portion being generated by splitting the second portion through the second optical filter, wherein the spectroscopic unit may receive a fourth portion of the welding light, the fourth portion being generated by splitting the second portion through the second optical filter.

According to one or more embodiments, a wavelength band of the third portion may be determined based on a type of welding target.

According to one or more embodiments, the infrared sensor may perform a high dynamic range (HDR) processing on the first portion, and the visible light sensor may perform the HDR processing on the third portion.

To solve the above technical problem, a method for training a machine learning model for welding defect inspection according to an embodiment of the present disclosure may include generating training data including reference optical measurement data measured using an optical device during welding of a reference weldment based on a plurality of welding conditions, and training a machine learning model for determining an occurrence of welding defects in a target weldment using the training data, wherein the optical device may include an infrared detection unit that detects heat of a weldment, a visible light detection unit that detects plasma of the weldment, and a spectroscopic unit that detects wavelength-specific luminosity of the weldment.

According to one or more embodiments, each of the plurality of welding conditions may be a condition that varies at least one of an output power of a welding laser, a focal point of the welding laser, or a spacing between weld targets of the weldment.

According to one or more embodiments, at least some of the plurality of welding conditions may be normal welding conditions determined in advance of welding.

According to one or more embodiments, the reference optical measurement data may include reference thermal data detected using the infrared detection unit, reference plasma data detected using the visible light detection unit, and reference wavelength-specific luminosity data detected using the spectroscopic unit, wherein the reference thermal data, the reference plasma data, and the reference wavelength-specific luminosity data may be detected during welding of the reference weldment under each of the plurality of welding conditions.

According to one or more embodiments, the reference thermal data may include information associated with a contour extracted based on infrared intensity from an infrared image generated using the infrared detection unit during welding of the reference weldment.

According to one or more embodiments, the reference thermal data may include at least one of shape information, temperature information, or luminance information of the contour.

According to one or more embodiments, the reference thermal data may include information associated with a hot spot, which is a point of maximum infrared intensity in an infrared image generated using the infrared detection unit during welding of the reference weldment, and the information associated with the hot spot may include at least one of brightness information of a region of the hot spot or positional information of the hot spot in the infrared image.

According to one or more embodiments, the generating of the training data may include obtaining bead information associated with a bead formed at a reference weldment where welding has been completed under each of the plurality of welding conditions and including the bead information in the training data, wherein the bead information may include at least one of length information of the bead, depth information of the bead, or surface defect information of the bead.

According to one or more embodiments, the machine learning model may determine the occurrence of welding defects in the target weldment based on optical measurement data measured using the optical device during welding of the target weldment.

According to one or more embodiments, the optical measurement data measured during welding of the target weldment may include thermal data of the target weldment detected using the infrared detection unit, plasma data of the target weldment detected using the visible light detection unit, and wavelength-specific luminosity data of the target weldment detected using the spectroscopic unit, wherein the thermal data, the plasma data, and the wavelength-specific luminosity data may be detected during welding of the target weldment.

According to one or more embodiments, the machine learning model may determine a first occurrence of a first defect type indicating defects associated with the welding conditions, a second occurrence of a second defect type indicating defects associated with a length of a bead or a depth of the bead, and/or a third occurrence of a third defect type indicating defects associated with surface defects of the bead.

According to one or more embodiments, the machine learning model may be trained to correlate the reference optical measurement data with at least one of the welding conditions, the length information of the bead, the depth information of the bead, and/or the surface defect information of the bead, based on the input training data.

A non-transitory computer-readable storage medium storing instructions for executing the method for training a machine learning model for welding defect inspection may be provided.

To solve the above technical problem, a computing apparatus may include a memory (e.g., non-transitory memory), and at least one processor connected to the memory and configured to execute at least one computer-readable program included in the memory, wherein the at least one computer-readable program may include instructions to generate training data including reference optical measurement data measured using an optical device during welding of a reference weldment based on a plurality of welding conditions and to train a machine learning model for determining an occurrence of welding defects in a target weldment using the training data, wherein the optical device may include an infrared detection unit that detects heat of a weldment, a visible light detection unit that detects plasma of the weldment, and a spectroscopic unit that detects wavelength-specific luminosity of the weldment.

According to some embodiments of the present disclosure, the welding defect inspection can be performed in less time without destroying an inspection target object.

According to some embodiments of the present disclosure, a passband of an optical filter is determined to be a wavelength band over which the amount of change in an image is equal to or greater than a threshold, so that light in the wavelength band that best represents a welding state is imaged. Thus, the welding characteristics can be clearly represented in an image included in training data of a machine learning model. Accordingly, the performance of the trained machine learning model may be improved.

However, aspects and features of the present disclosure are not limited to those described above, and other aspects and features not mentioned will be clearly understood by a person skilled in the art from the detailed description, described below.

### BRIEF DESCRIPTION OF THE DRAWINGS

The following drawings attached to this specification illustrate embodiments of the present disclosure, and further describe aspects and features of the present disclosure together with the detailed description of the present disclosure. Thus, the present disclosure should not be construed as being limited to the drawings:
FIG. 1 illustrates an optical device and a machine learning model for inspecting welding defects, according to some embodiments of the present disclosure;
FIG. 2 illustrates an example of an optical device according to one embodiment of the present disclosure;
FIG. 3 illustrates a training process of a machine learning model according to one embodiment of the present disclosure;
FIG. 4 illustrates images of a reference weldment under a plurality of different welding conditions according to one embodiment of the present disclosure;
FIG. 5 illustrates an example of reference thermal data according to one embodiment of the present disclosure;
FIG. 6 illustrates a cross-sectional view of a bead formed at a reference weldment after completion of welding according to one embodiment of the present disclosure;
FIG. 7 illustrates an inference process of a machine learning model according to one embodiment of the present disclosure;
FIG. 8 illustrates an example of a machine learning model according to some embodiments of the present disclosure;
FIG. 9 illustrates an artificial neural network model according to one embodiment of the present disclosure; and
FIG. 10 illustrates a method for training a machine learning model for welding defect inspection according to one embodiment of the present disclosure.

### DETAILED DESCRIPTION

Hereinafter, embodiments of the present disclosure will be described, in detail, with reference to the accompanying drawings. The terms or words used in the present specification and claims are not to be limitedly interpreted as general or dictionary meanings and should be interpreted as meanings and concepts that are consistent with the technical idea of the present disclosure on the basis of the principle that an inventor can be his/her own lexicographer to appropriately define concepts of terms to describe his/her invention in the best way.

The embodiments described in this specification and the configurations shown in the drawings are only some of the embodiments of the present disclosure and do not represent all of the technical aspects, and features of the present disclosure. Accordingly, it should be understood that there may be various equivalents and modifications that can replace or modify the embodiments described herein at the time of filing this application.

It will be understood that when an element or layer is referred to as being "on," "connected to," or "coupled to" another element or layer, it may be directly on, connected, or coupled to the other element or layer or one or more intervening elements or layers may also be present. When an element or layer is referred to as being "directly on," "directly connected to," or "directly coupled to" another element or layer, there are no intervening elements or layers present. For example, when a first element is described as being "coupled" or "connected" to a second element, the first element may be directly coupled or connected to the second element or the first element may be indirectly coupled or connected to the second element via one or more intervening elements.

In the figures, dimensions of the various elements, layers, etc. may be exaggerated for clarity of illustration. The same reference numerals designate the same elements. As used herein, the term "and/or" includes any and all combinations of one or more of the associated listed items. Further, the use of "may" when describing embodiments of the present disclosure relates to "one or more embodiments of the present disclosure." Expressions, such as "at least one of" and "any one of," when preceding a list of elements, modify the entire list of elements and do not modify the individual elements of the list. When phrases such as "at least one of A, B and C, "at least one of A, B or C," "at least one selected from a group of A, B and C," or "at least one selected from among A, B and C" are used to designate a list of elements A, B and C, the phrase may refer to any and all suitable combinations or a subset of A, B and C, such as A, B, C, A and B, A and C, B and C, or A and B and C. As used herein, the terms "use," "using," and "used" may be considered synonymous with the terms "utilize," "utilizing," and "utilized," respectively. As used herein, the terms "substantially," "about," and similar terms are used as terms of approximation and not as terms of degree, and are intended to account for the inherent variations in measured or calculated values that would be recognized by those of ordinary skill in the art.

It will be understood that, although the terms first, second, third, etc. may be used herein to describe various elements, components, regions, layers, and/or sections, these elements, components, regions, layers, and/or sections should not be limited by these terms. These terms are used to distinguish one element, component, region, layer, or section from another element, component, region, layer, or section. Thus, a first element, component, region, layer, or section discussed below could be termed a second element, component, region, layer, or section without departing from the teachings of example embodiments.

Spatially relative terms, such as "beneath," "below," "lower," "above," "upper," and the like, may be used herein for ease of description to describe one element or feature's relationship to another element(s) or feature(s) as illustrated in the figures. It will be understood that the spatially relative terms are intended to encompass different orientations of the device in use or operation in addition to the orientation depicted in the figures. For example, if the device in the figures is turned over, elements described as "below" or "beneath" other elements or features would then be oriented "above" or "over" the other elements or features. Thus, the term "below" may encompass both an orientation of above and below. The device may be otherwise oriented (rotated 90 degrees or at other orientations), and the spatially relative descriptors used herein should be interpreted accordingly.

The terminology used herein is for the purpose of describing embodiments of the present disclosure and is not intended to be limiting of the present disclosure. As used herein, the singular forms "a" and "an" are intended to include the plural forms as well, unless the context clearly indicates otherwise. It will be further understood that the terms "includes," "including," "comprises," and/or "comprising," when used in this specification, specify the presence of stated features, integers, steps, operations, elements, and/or components but do not preclude the presence or addition of one or more other features, integers, steps, operations, elements, components, and/or groups thereof.

Also, any numerical range disclosed and/or recited herein is intended to include all sub-ranges of the same numerical precision subsumed within the recited range. For example, a range of "1.0 to 10.0" is intended to include all subranges between (and including) the recited minimum value of 1.0 and the recited maximum value of 10.0, that is, having a minimum value equal to or greater than 1.0 and a maximum value equal to or less than 10.0, such as, for example, 2.4 to 7.6. Any maximum numerical limitation recited herein is intended to include all lower numerical limitations subsumed therein, and any minimum numerical limitation recited in this specification is intended to include all higher numerical limitations subsumed therein. Accordingly, Applicant reserves the right to amend this specification, including the claims, to expressly recite any sub-range subsumed within the ranges expressly recited herein. All such ranges are intended to be inherently described in this specification such that amending to expressly recite any such subranges would comply with the requirements of 35 U.S.C. § 112(a) and 35 U.S.C. § 132(a).

References to two compared elements, features, etc. as being "the same" may mean that they are "substantially the same". Thus, the phrase "substantially the same" may include a case having a deviation that is considered low in the art, for example, a deviation of 5% or less. In addition, when a certain parameter is referred to as being uniform in a given region, it may mean that it is uniform in terms of an average.

Throughout the specification, unless otherwise stated, each element may be singular or plural.

Arranging an arbitrary element "above (or below)" or "on (under)" another element may mean that the arbitrary element may be disposed in contact with the upper (or lower) surface of the element, and another element may also be interposed between the element and the arbitrary element disposed on (or under) the element.

In addition, it will be understood that when a component is referred to as being "linked," "coupled," or "connected" to another component, the elements may be directly "coupled," "linked" or "connected" to each other, or another component may be "interposed" between the components".

Throughout the specification, when "A and/or B" is stated, it means A, B or A and B, unless otherwise stated. That is, "and/or" includes any or all combinations of a plurality of items enumerated. When "C to D" is stated, it means C or more and D or less, unless otherwise specified.

In the present disclosure, "welding light" may refer to light generated during welding of a target object. The "welding light" is not limited to light in a range of the visible wavelengths, but may also refer to light in a range of the infrared wavelengths and/or light in a range of ultraviolet wavelengths.

Further, in the present disclosure, "luminosity" may refer to the intensity of light, but is not limited to referring to the intensity of light in the range of the visible wavelengths. For example, the "luminosity" may be used to refer to the intensity of light in the range of the infrared wavelengths and/or the range of ultraviolet wavelengths.

Further, in the present disclosure, "contour" may refer to the outline or interior region of a contour within an image or refer to the actual contour or interior region of a contour in a physical space corresponding to the contour within the image.

As described herein, laser welding is widely employed in a production process of secondary batteries, and various inspection methods are used to measure the quality of a weldment made through the laser welding. For example, there may be provided destructive inspection methods for measuring a tensile strength of a weldment or a depth of penetration after cutting a cross-section of the weld weldment, as well as non-destructive inspection methods such as using X-rays. However, these methods have problems such as requiring a long time for inspection or destroying a target object, and thus the inspection is limited to sampling. Therefore, the inventors have identified that it is necessary to introduce a weldment inspection method to solve these problems.

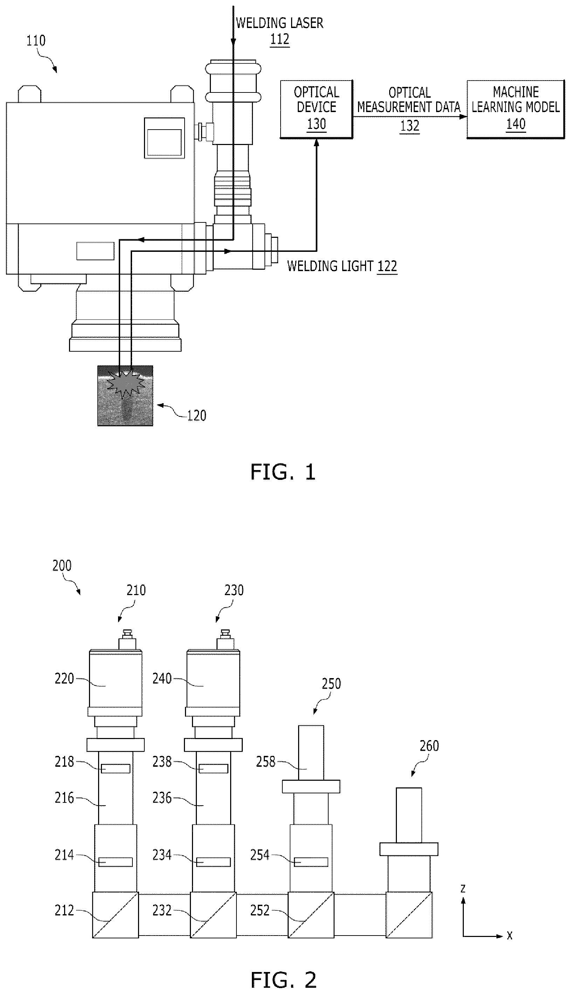

FIG. 1 is a diagram illustrating a system including an optical device 130 and a machine learning model 140 (e.g., executing on a computing apparatus) for inspecting welding defects according to some embodiments of the present disclosure. A welding device 110 may be provided to weld a weldment 120 of a welding target. The weldment 120 may refer to a portion where two or more welding targets are welded to be joined together. The welding device 110 is configured to weld the weldment 120 by irradiating the weldment 120 with a welding laser 112 to melt (fuse) a weld metal. The machine learning model may be implemented as a MultiLayer Perceptron (MLP). Alternatively, a Convolutional Neural Network (CNN) or a Recurrent Neural Network (RNN) can be selected for similar applications. Here, the ReLU (Rectified Linear Unit) activation function may be primarily used. For the output layer, Sigmoid may be chosen for binary classification and Softmax may be chosen for multi-class classification. As input, based on optical data (e.g., infrared data, visible light data, wavelength-specific intensity data), 3 to 10 key features may be extracted. Depending on the situation and the need to handle high-resolution data, the feature set can be expanded to 20-50 features. As input data, infrared images, temperature information extracted from these images, brightness information, hotspot location data, etc., plasma data obtained from visible light imagery, etc., wavelength-specific intensity data obtained from spectroscopic analysis, etc. may be used. The training dataset may consist of approximately 1,000 to 10,000 welding cases. The ratio of normal to defective welds may be balanced at about 1:1. As hidden layers, typically 2-5 hidden layers can be used. For more complex data, this can be extended to 6-10 hidden layers. Each hidden layer can contain between 64 and 256 nodes, adjustable based on problem complexity and data size. For binary classification: 1 output (indicating defect presence: 0 or 1) can be get and for multi-class classification (e.g., 3 types of defects): 3 outputs can be get. For binary classification: defect status information (0: normal, 1: defective) and for multi-class classification: probability values for defect types 1, 2, and 3.can be use.

While the weldment 120 is welded by the welding device 110, welding light 122 may be generated at the weldment 120. The optical device 130 may be provided to detect the welding light 122 generated at the weldment 120. The optical device 130 may include an infrared detection unit that detects heat generated at the weldment 120 by detecting light in the infrared wavelength range, a visible light detection unit that detects plasma generated at the weldment 120 by detecting light in the visible wavelength range, and/or a spectroscopic unit that detects wavelength-specific luminosity at the weldment 120. The detailed configuration of the optical device 130 will be described further with reference to FIG. 2.

The optical device 130 may generate and output optical measurement data 132 as a result of detecting the welding light 122. For example, the optical measurement data 132 may include information related to heat, plasma, and/or wavelength-specific luminosity generated at the weldment 120.

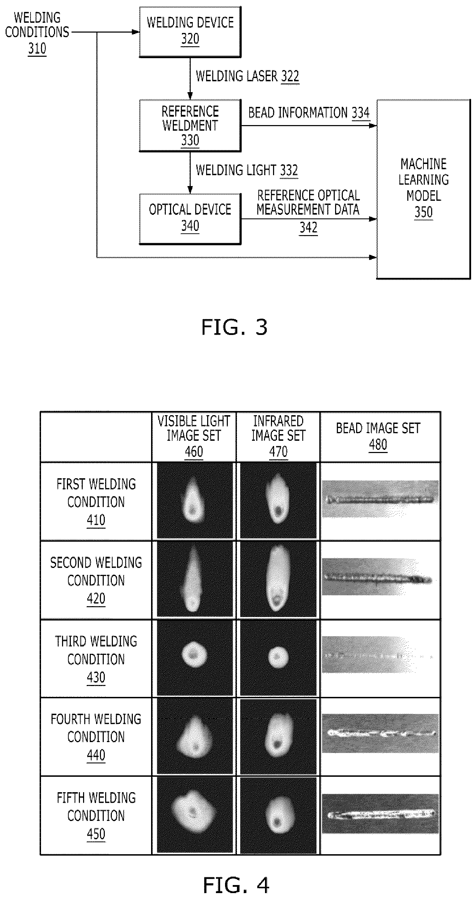

The optical measurement data 132 may be used in the training and inference process of the machine learning model 140. In the training phase of the machine learning model 140, the machine learning model 140 may be trained using reference optical measurement data generated by the optical device 130 based on welding light generated during welding of a reference weldment. This process will be described in more detail with reference to FIG. 3.

Additionally, the machine learning model 140 may be further trained using bead information associated with a bead formed at the reference weldment 330 where welding has been completed. For example, the bead information may include bead length information, bead depth information, or bead surface defect information.

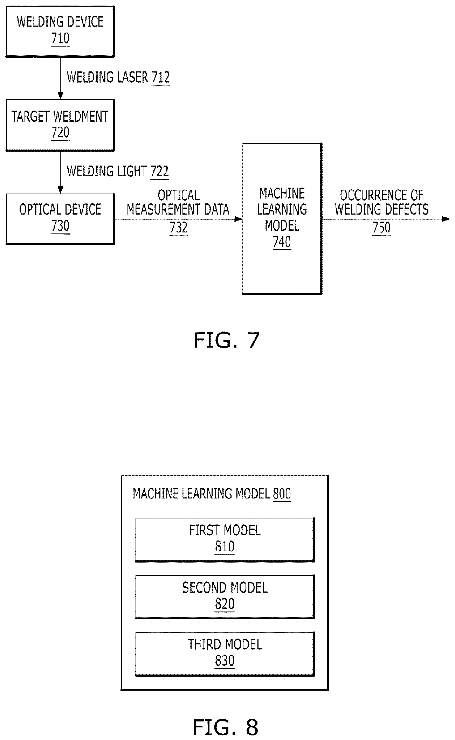

Once the machine learning model 140 has been trained, the trained machine learning model 140 may receive optical measurement data generated by the optical device 130 based on welding light emitted during welding of a target weldment that is subject to determination of the occurrence of welding defects and output information associated with the occurrence of welding defects. This process will be described later with reference to FIG. 7.

FIG. 2 is a diagram illustrating an example of an optical device 200 according to one embodiment of the present disclosure. The optical device 200 may be optical device 130 shown in FIG. 1, in some embodiments. The optical device 200 may include an infrared detection unit 210, a visible light detection unit 230, a spectroscopic unit 250, and a monitor 260. Based on the heat, plasma, and luminosity of a weldment detected by the optical device 200, the occurrence of welding defects at the weldment may be determined.

In one embodiment, welding light generated during welding of the weldment (e.g., welding light 122) may pass through the infrared detection unit 210, the visible light detection unit 230, the spectroscopic unit 250, and the monitor 260 in sequence. In FIG. 2, it is shown that the welding light proceeds in a direction (+x direction) from the left side of the infrared detection unit 210 toward the monitor 260. Without being limited thereto, the optical device 200 may include additional components, and the connection order of these components is not limited to the connection order shown in FIG. 2.

In one embodiment, each of the infrared detection unit 210, the visible light detection unit 230, the spectroscopic unit 250, and monitor 260 may be modularized, and one or more of the infrared detection unit 210, the visible light detection unit 230, the spectroscopic unit 250, and monitor 260 may be combined in any order to be included in the optical device 200. In another embodiment, the optical device 200 may include a configuration where the infrared detection unit 210, the visible light detection unit 230, the spectroscopic unit 250, and monitor 260 are connected in sequence without the spectroscopic unit 250.

The infrared detection unit 210 may detect heat generated from the weldment being welded and visualize (e.g., generate) an image of the generated heat. The infrared detection unit 210 may include a first optical filter 212, a first focusing lens 214, an optical tube 216, a second optical filter 218, and an infrared sensor 220.

The first optical filter 212 may reflect or transmit a portion of the welding light generated during welding of the weldment. For example, the first optical filter 212 may serve as a dichroic mirror and may reflect a wavelength band equal to or greater than a specific wavelength value (e.g., 1000 nm) of the welding light and transmit a wavelength band below a specific wavelength value of the welding light. A portion of the welding light reflected by the first optical filter 212 (e.g., a wavelength band included in the infrared band) may be transmitted to the infrared sensor 220 while a portion of the welding light transmitted by the first optical filter 212 (e.g., a wavelength band included in the visible light band) may be transmitted to the visible light detection unit 230 (or, alternatively or additionally, to a third optical filter 232). Therefore, the infrared sensor may receive a portion of the welding light reflected from the first optical filter.

The first focusing lens 214 may focus a portion of the welding light reflected from the first optical filter 212. For example, the first focusing lens 214 may have a focal length of 200 mm. The optical tube 216 may be configured to allow a length adjustment in a z-axis direction to precisely adjust the focal length of the first focusing lens 214.

The second optical filter 218 may allow only a specific band of the portion of the welding light that has passed through the first focusing lens 214 to pass therethrough. For example, the second optical filter 218 may be a bandpass filter.

The infrared sensor 220 may collect (e.g., may receive) the portion of the welding light that has passed through the second optical filter 218. The band of the welding light that can be collected and detected by the infrared sensor 220 may correspond to the short wavelength infrared (SWIR) band with a wavelength of 1000 nm to 2000 nm. Accordingly, the infrared sensor 220 may have characteristics suitable for imaging and representing heat components near the melting points of aluminum (Al) and copper (Cu), such as approximately 660°C and 1085°C, respectively, which are commonly used as welding samples for secondary batteries.

The infrared sensor 220 may perform a high dynamic range (HDR) processing on a portion of the collected welding light (e.g., received by the infrared sensor). The welding light that is not HDR processed may cause saturation of a typical complementary metal oxide semiconductor (CMOS) sensor, resulting in an inability to identify meaningful brightness information for image analysis. By applying HDR processing to the portion of the collected welding light, the infrared sensor 220 may clarify brightness differences between regions in the output infrared image to derive meaningful brightness information for image analysis.

The infrared sensor 220 may be moved in an x-axis direction and/or a y-axis direction (e.g., out-of-the-page direction in FIG. 2) for center alignment of the infrared sensor 220.

In one embodiment, the wavelength band of the portion of the welding light collected by the infrared sensor 220 may be determined based on the type of welding sample and/or welding target being used. For example, the reflection/transmission bands of the first optical filter 212, the focal length of the first focusing lens 214, and/or the passband of the second optical filter 218 may be determined based on the type of welding sample and/or welding target being used.

The visible light detection unit 230 may detect plasma generated from and/or at the weldment and visualize (e.g., generate) an image of the generated plasma. The visible light detection unit 230 may include the third optical filter 232, a second focusing lens 234, an optical tube 236, a fourth optical filter 238, and a visible light sensor 240.

The third optical filter 232 may split the light collected (e.g., may receive a portion of light and split such a portion) after passing through (e.g., transmitting through) the first optical filter 212. For example, the third optical filter 232 may be a beam splitter. The third optical filter 232 may transmit a portion of the collected light (e.g., 50% of the light) to the visible light sensor 240 and transmit the remaining portion to the spectroscopic unit 250 (or, alternatively or additionally, to a fifth optical filter 252).

The second focusing lens 234 may focus a portion of the welding light split by the third optical filter 232. For example, the second focusing lens 234 may have a focal length of 200 mm. The optical tube 236 may be configured to allow a length adjustment in the z-axis direction to precisely adjust the focal length of the second focusing lens 234.

The fourth optical filter 238 may allow only a specific band of the portion of the welding light that has passed through the second focusing lens 234 to pass therethrough. The fourth optical filter 238 may be a bandpass filter.

The visible light sensor 240 may collect a portion of the welding light that has passed through the fourth optical filter 238. For example, the portion of the welding light that can be collected by the visible light sensor 240 may fall in a band of wavelengths between 400 nm and 800 nm. Accordingly, the visible light sensor 240 may have characteristics suitable for imaging and representing light components near the melting points of aluminum and copper (approximately 660 °C and 1085 °C, respectively), which are commonly used as weld samples for secondary batteries.

The visible light sensor 240 may perform the HDR processing on a portion of the collected welding light. The portion may be the portion generated by splitting a portion through the third optical filter. The visible light sensor 240 may be moved in the x-axis direction and/or the y-axis direction for center alignment of the visible light sensor 240.

In one embodiment, the wavelength band of the portion of the welding light collected by the visible light sensor 240 may be determined based on the type of welding sample and/or welding target being used. For example, the focal length of the second focusing lens 234 and/or the passband of the fourth optical filter 238 may be determined based on the type of welding sample and/or welding target being used.

The spectroscopic unit 250 may include the fifth optical filter 252, a third focusing lens 254, and a spectrometer 258.

The fifth optical filter 252 may split the light collected (e.g., received) after passing through the third optical filter 232. For example, the fifth optical filter 252 may be a beam splitter. The fifth optical filter 252 may transmit a portion of the collected light (e.g., 50% of the collected light) to the spectrometer 258 and transmit the remaining portion to the monitor 260.

The third focusing lens 254 may focus a portion of the welding light split by the fifth optical filter 252. For example, the third focusing lens 254 may have a focal length of 100 mm.

The spectrometer 258 may detect the wavelength-specific luminosity of and/or at the weldment. For example, the spectrometer 258 may output a luminosity spectrum in a wavelength band of 200 nm to 1000 nm.

The monitor 260 may receive the portion of the light split by the fifth optical filter 252. The monitor 260 may be used for sample verification prior to welding.

The passband of the second optical filter 218 and/or the passband of the fourth optical filter 238 may be determined to be a wavelength band over which the amount of change in an acquired image as an output power of the welding laser is varied is equal to or greater than a threshold. For example, the second optical filter 218 may be configured to pass a portion of the welding light in the 1400±20 nm band while the fourth optical filter 238 may be configured to pass a portion of the welding light in the 500±20 nm band. This can ensure, in some embodiments, that light in the wavelength band that best represents a welding state is imaged, so that welding characteristics can be clearly represented in the image.

FIG. 3 is a diagram illustrating a training process of a machine learning model 350 according to one embodiment of the present disclosure. A reference weldment 330 may refer to a weldment that is welded to generate training data for the machine learning model 350, and reference optical measurement data 342 may refer to data generated based on the welding light generated by the welding of the reference weldment 330. In other words, corresponding to the term "X" or the term "target X" used in the inference process, the terms used in the training process may be defined as "reference X."

A welding device 320 may weld the reference weldment 330 based on a plurality of welding conditions 310. For example, each of the welding conditions 310 may be a condition that varies including at least one of an output power of the welding laser 322, a focal point of the welding laser 322, or a spacing between weld targets of the weldment. At least some of the welding conditions 310 may be normal (e.g., standard) welding conditions determined in advance. The plurality of welding conditions 310 will be described in more detail with reference to FIG. 4.

The welding conditions 310, bead information 334 of the reference weldment 330, and/or the reference optical measurement data 342 generated from an optical device 340 may be input to the machine learning model 350 as training data to train the machine learning model 350 for determining the occurrence of welding defects in the target weldment.

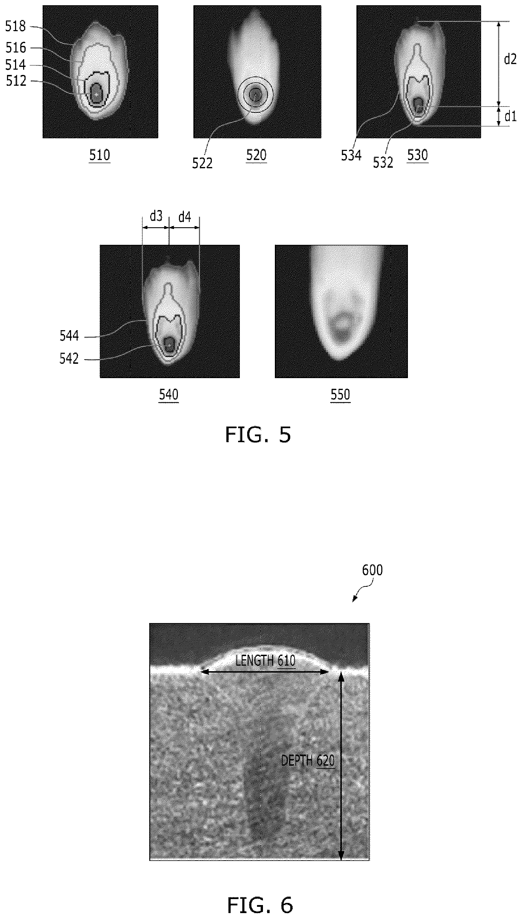

The bead information 334 of the reference weldment 330 where welding has been completed under each of the plurality of welding conditions 310 may include at least one of bead length information, bead depth information, or bead surface defect information. The bead length information and the bead depth information will be discussed further with reference to FIG. 6. The bead surface defect information may include information about the occurrence of defects such as bead pinholes, scorching, and the like.

As the welding device 320 welds the reference weldment 330 based on the plurality of welding conditions, welding light 332 may be generated during welding of the reference weldment 330. The welding light 332 may be detected by the optical device 340, and the optical device 340 may generate and output the reference optical measurement data 342. The optical device 340 may correspond to the optical device 200 shown in FIG. 2, the optical device 130 shown in FIG. 1, and the description of the optical device 200 shown in FIG. 2 and optical device 130 shown in FIG. 1 can be applicable to the optical device 340. Thus, a redundant description thereof will be omitted.

The reference optical measurement data 342 may include reference thermal data detected using the infrared detection unit of the optical device, reference plasma data detected using the visible light detection unit of the optical device, and reference wavelength-specific luminosity data detected using the spectroscopic unit of the optical device. The reference thermal data, the reference plasma data, and/or the reference wavelength-specific luminosity data are detected during welding of the reference weldment 330 under each of the plurality of welding conditions, in some embodiments.

The reference thermal data and the reference plasma data may include images (e.g., in a stacked image format, such as a tag image file format (Tiff)) of the reference weldment during welding and/or data from processing and/or analyzing the images. For example, the reference thermal data may include an infrared image of the weldment during welding and/or data from processing and/or analyzing the infrared image. Similarly, the reference plasma data may include a visible light image of the weldment during welding and/or data from processing and/or analyzing the visible light image. Further, the reference thermal data and the reference plasma data may include a plurality of images or a stack of such images taken at predetermined time intervals.

The reference thermal data may include information associated with a contour extracted based on infrared intensity from an infrared image generated and/or acquired using the infrared detection unit during welding of the reference weldment. Additionally or alternatively, the reference thermal data may include information associated with a hot spot, which is a point of maximum infrared intensity in the infrared image generated using the infrared detection unit during welding of the reference weldment. This will be described in more detail with reference to FIG. 5.

The reference wavelength-specific luminosity data may include wavelength-specific luminosity spectrum data measured at regular time intervals (e.g., every 0.04 seconds) during welding of the reference weldment 330. In one embodiment, the reference wavelength-specific luminosity data may be represented as luminosity (light intensity) at specific wavelengths at a particular time point or luminosity variation over time for a particular wavelength range.

The machine learning model 350 may be trained to correlate the reference optical measurement data 342 with at least one of the welding conditions 310, the length information of the bead, the depth information of the bead, and/or the surface defect information of the bead, based on the input training data. The machine learning model of FIG. 3 may be representative of a portion of a computing apparatus that includes a memory including at least one computer-readable program as described herein.

FIG. 4 is a diagram illustrating images of a reference weldment under a plurality of different welding conditions including first welding condition 410, second welding condition 420, third welding condition 430, fourth welding condition 440, and fifth welding condition 450, according to at least one embodiment of the present disclosure. A visible light image set 460 may be generated by a visible light detection unit of an optical device during welding of the reference weldment, and an infrared image set 470 may be generated by an infrared detection unit of the optical device during welding of the reference weldment. A bead image set 480 may include images of the beads captured after welding of the reference weldment has been completed. The visible light image set 460, the infrared image set 470, and/or the bead image set 480 may be utilized as training data for training a machine learning model that can be used to determine the occurrence of welding defects in the target weldment.

Each of the welding conditions, first to fifth welding conditions 410, 420, 430, 440, and 450, may be a condition that varies at least one of an output power of the welding laser, a focal point of the welding laser, and/or a spacing between weld targets of the weldment. At least some of the welding conditions, first to fifth welding conditions 410, 420, 430, 440, and 450, may be normal (e.g., standard) welding conditions determined in advance. For example, each of the welding conditions, first to fifth welding conditions 410, 420, 430, 440, and 450, may be a condition where the output power of the welding laser is increased (e.g., over-welding) or decreased (e.g., under-welding) relative to the normal output power. In another example, each of the welding conditions, first to fifth welding conditions 410, 420, 430, 440, and 450, may be a condition where the focal length is lengthened or shortened relative to the normal focal length. Further, each of the welding conditions, first to fifth welding conditions 410, 420, 430, 440, and 450, may be a condition where the amount of shielding gas, welding speed, and/or level of contamination on the welding surface varies from one another.

Specifically, the first welding condition 410 may be the normal condition. A visible light image, an infrared image, and a bead image corresponding to the first welding condition 410 may be utilized as reference images.

The second welding condition 420 may be a condition in which the output power of the welding laser is increased compared to the first welding condition 410. In a visible light image and an infrared image corresponding to the second welding condition 420, compared to the first welding condition 410, a thermal trace may appear longer, a penetration hole may be observed, and an explosion may appear at the pinhole.

The third welding condition 430 may be a condition in which the output power of the welding laser is reduced compared to the first welding condition 410. In a visible light image and an infrared image corresponding to the third welding condition 430, the central luminosity may be reduced, and the welding light may be generated in a circular shape rather than an elliptical shape.

The fourth welding condition 440 may be a condition in which a focal length of the welding laser is longer compared to the first welding condition 410. In a visible light image and an infrared image corresponding to the fourth welding condition 440, the central luminosity is similar to that in the image of the normal condition, but the welding light is generated in a broadened shape in the width direction.

The fifth welding condition 450 may be a condition in which a focal length of the welding laser is shorter compared to the first welding condition 410. In a visible light image and an infrared image corresponding to the fifth welding condition 450, the central luminosity is similar to that in the image of the normal condition, but the welding light is more circular.

The visible light image set 460, the infrared image set 470, and the bead image set 480 for different welding conditions described with reference to FIG. 4 are merely examples, and a machine learning model may be trained based on the visible light image set 460, the infrared image set 470, and the bead image set 480 for different welding conditions.

FIG. 5 is a diagram illustrating an example of reference thermal data according to one embodiment of the present disclosure. The reference thermal data may include information associated with contours 512, 514, 516, and 518 that are extracted based on infrared intensity from an infrared image generated using an infrared detection unit during welding of the reference weldment. The contours 512, 514, 516, and 518 may be determined based on the infrared intensity in the infrared image. For example, each of the contours 512, 514, 516, and 518 may be a contour line connecting points having the luminosity of a predefined arbitrary proportion (e.g., 80%, 60%, 40%, and 5%) of the luminosity of a point having the highest energy in the infrared image. In FIG. 5, four contours 512, 514, 516, and 518 are shown in a first infrared image 510. However, without being limited thereto, any arbitrary number of contours may be determined.

The reference thermal data may include at least one of shape information, temperature information, or luminance information of the contours 512, 514, 516, and 518. For example, the reference thermal data may include the shape information of each of the contours 512, 514, 516, and 518 such as an area, a perimeter, a width, a length, an aspect ratio, and the like. Further, the reference thermal data may include the temperature information or the luminance information of each of the contours 512, 514, 516, and 518 such as an average brightness, maximum brightness, and the like.

Additionally or alternatively, the reference thermal data may include information associated with a hot spot, which is a point of maximum infrared intensity in the infrared image generated using the infrared detection unit during welding of the reference weldment. The information associated with the hot spot may include at least one of brightness information of the hot spot region or positional information of the hot spot in the infrared image.

In one example, the brightness information of the hot spot region may include information associated with the brightness of a hot spot 522 in a second infrared image 520 and/or the average brightness of concentric circular regions surrounding the hot spot 522.

In one example, the positional information of the hot spot may include information associated with a ratio of an upper portion to a lower portion of the welding light relative to the hot spot. For example, the information associated with the hot spot may include information associated with a ratio of an upper portion to a lower portion (e.g., with reference to distances d1 and d2, d2/d1 or d1/d2) of the outermost contour 534 relative to a hot spot 532 in a third infrared image 530. Therefore, the level of molten pool formation at the weldment may be represented, and the distinction between under-welding and over-welding conditions may be facilitated by a trained machine learning model.

In one example, the positional information of the hot spot may include information associated with a ratio of a left portion to a right portion of the welding light relative to the hot spot. For example, the information associated with the hot spot may include information associated with a ratio of a left portion to a right portion (e.g., with reference to distances d3 and d4, d4/d3 or d3/d4) of the outermost contour 544 relative to a hot spot 542 in a fourth infrared image 540. This may facilitate the distinction between over-welding and focus-defect conditions using a machine learning model.

In one example, the information associated with the hot spot may include information associated with the through-hole (e.g., size of the through-hole), which refers to a hole with low luminosity at the hot spot position. Since through-holes are commonly observed during over-welding, for example, due to damage to the separator, machine learning models may be utilized to facilitate the identification of over-welding conditions.

FIG. 5 also shows a fifth infrared image 550.

FIG. 6 is a diagram illustrating a cross-sectional view of a bead 600 formed at a reference weldment after completion of welding, according to one embodiment of the present disclosure. Bead information associated with a bead formed at the reference weldment 330 where welding has been completed under each of the plurality of welding conditions (e.g., as described in relation to FIG. 4) may include information associated with a length 610 of a bead 600 and/or information associated with a depth 620 of the bead 600.

The bead information may be input to a machine learning model as training data for the machine learning model. The bead information may be provided as data indicative of bead information as described herein.

FIG. 7 is a diagram illustrating an inference process of a machine learning model 700 according to one embodiment of the present disclosure. A welding device 710 and an optical device 730 may correspond to the welding device 320 and the optical device 340 shown in FIG. 3 and/or the optical device of FIGS. 1 and 2, and the descriptions of the welding device 320 and the optical device 340 shown in FIG. 3 (and/or FIGS. 1 and 2) can be applicable to the welding device 710 and the optical device 730. Thus, a redundant description thereof will be omitted.

The welding device 710 may irradiate a target weldment 720, which is subject to determination of the occurrence of welding defects, with a welding laser 712 according to an arbitrary welding condition. Welding light 722 generated when the welding device 710 welds the target weldment 720 is detected by the optical device 730, and the optical device 730 generates and outputs optical measurement data 732 indicating the detection result, in some embodiments.

In one embodiment, the optical measurement data 732 input to a trained machine learning model 740 may include thermal data of the target weldment 720 detected using an infrared detection unit of the optical device 730, plasma data of the target weldment 720 detected using a visible light detection unit of the optical device 730, and/or wavelength-specific luminosity data of the target weldment 720 detected using a spectroscopic unit of the optical device 730. The thermal data, the plasma data, and the wavelength-specific luminosity data may be detected during welding of the target weldment 720.

The machine learning model 740, which is trained as described in FIGS. 3 to 6, may output information associated with the occurrence of welding defects 750 in the target weldment 720 (that is, whether welding defects have occurred in the target weldment) based on the optical measurement data 732. For example, the machine learning model 740 may determine the occurrence of different defect types such as a first defect type indicating defects associated with welding conditions, a second defect type indicating defects associated with the length or depth of the bead, and a third defect type indicating defects associated with surface defects of the bead. In one embodiment, the machine learning model 740 includes a plurality of models, and the different defect types may be determined using each of the plurality of models, respectively. This will be discussed further with reference to FIG. 8.

In one example, the trained machine learning model 740 may infer whether a welding condition, such as an output power of the welding laser 712, a focal point of the welding laser 712, and/or a spacing between weld targets of the weldment, is out of the normal range based on the optical measurement data 732 (e.g., inference of the first defect type).

In one example, the trained machine learning model 740 may numerically calculate the length of the bead and/or the depth of the bead on a bottom surface of the weldment based on the optical measurement data 732, and infer whether the depth of the bead and/or the length of the bead is out of the normal range (e.g., inference of the second defect type).

In one example, the trained machine learning model 740 may infer the occurrence of defects associated with surface defects of the bead such as bead pinholes, scorching, and the like based on the optical measurement data 732 (e.g., inference of the third defect type).

The machine learning model 740 may determine the occurrence of defects and assess the degree of defects based on how much the welding conditions, the length of the bead, or the depth of the bead deviate from predetermined normal conditions. In one example, the machine learning model 740 may determine that no defect associated with the output power of the welding laser has occurred if the output power of the welding laser 712 deviates by less than ±5% from the predetermined normal condition. In contrast, the machine learning model 740 may determine that a defect associated with the output power of the welding laser has occurred if the output power of the welding laser 712 deviates by more than ±5% from the predetermined normal condition. For example, the machine learning model 740 may classify the occurrence of defects as a low-level defect if the output power of the welding laser 712 deviates by more than ±5% but less than ±10% from the normal condition. The machine learning model 740 may classify the occurrence of defects as a medium-level defect if the output power of the welding laser 712 deviates by more than ±10% but less than ±15% from the normal condition. The machine learning model 740 may classify the occurrence of defects as a high-level defect if the output power of the welding laser 712 deviates by more than ±15% from the normal condition. The numerical ranges described above are exemplary, and the numerical ranges for classifying the degree of defects and the normal condition for determining the occurrence of defects and/or the degree of defects may be determined based on the type of weldment and the like.

FIG. 8 is a diagram illustrating an example of a machine learning model 800 according to one embodiment of the present disclosure. The machine learning model 800 may include a first model 810, a second model 820, and a third model 830. The diagram of FIG. 8 may be representative of a portion of a computing apparatus that includes a memory including at least one computer-readable program as described herein.

The first model 810 may determine the occurrence of a first defect type indicating defects associated with welding conditions. The first model 810 may be trained to correlate the welding conditions with reference optical measurement data. After the first model 810 is trained, the first model 810 may receive optical measurement data during welding (e.g., during a welding process) of a target weldment to determine the occurrence of the defects associated with the weld conditions. The determination may occur in real-time and/or at a later time.

The second model 820 may determine the occurrence of a second defect type indicating defects associated with a length of a bead or a depth of the bead. The second model 820 may be trained to correlate the length information of the bead and the depth information of the bead with reference optical measurement data. After the second model 820 is trained, the second model 820 may receive optical measurement data during welding (e.g., during a welding process) of the target weldment to determine the occurrence of the defects associated with the length or depth of the bead.

The third model 830 may determine the occurrence of a third defect type indicating defects associated with surface defects of a bead. For example, the third model 830 may be trained to correlate the surface defect information of the bead with reference optical measurement data. After the third model 830 is trained, the third model 830 may receive optical measurement data during welding (e.g., during a welding process) of the target weldment to determine the occurrence of defects associated with the surface defects of the bead (e.g., pinhole, scorching, etc.).

In one example, the first model 810 and the third model 830 may each be a classification model, and the second model 820 may be a regression model. Additionally or alternatively, each of the first model 810, the second model 820, and the third model 830 may be implemented as an ensemble model.

In FIG. 8, the machine learning model 800 is shown to include a plurality of different models (first, second, and third models 810, 820, and 830), and internal configurations of the machine learning model 800 are described herein by function, but this is for illustrative purposes only and does not imply that the plurality of models (first, second, and third models 810, 820, and 830) are necessarily physically distinct.

The internal configurations of the machine learning model 800 shown and described in FIG. 8 are merely examples, and some embodiments may further include other configurations in addition to the above internal configurations, and/or some configurations may be omitted. For example, a single model may be implemented to perform the functions of the plurality of models (first, second, and third models 810, 820, and 830) described above, or if some of the internal configurations are omitted, another model may be configured to perform the functions of some of the omitted internal configurations.

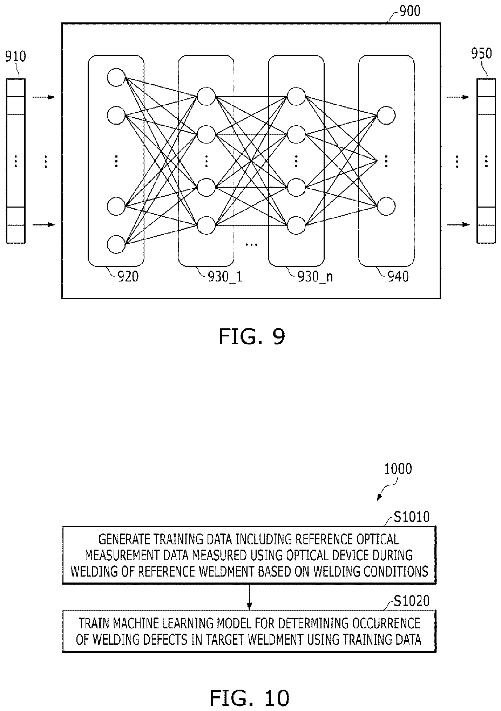

FIG. 9 is a diagram illustrating an artificial neural network model 900 according to one embodiment of the present disclosure. The artificial neural network model 900 is an example of a machine learning model, which is a statistical learning algorithm implemented based on machine learning techniques and the structure of biological neural networks, or a structure for implementing such an algorithm.

In one embodiment, the artificial neural network model 900 may represent a machine learning model with problem-solving capabilities in which nodes, which are artificial neurons forming a network through synaptic connections as in the biological neural network, are trained to iteratively adjust synaptic weights so that the error between the correct output and the inferred output in response to a given input is reduced. For example, the artificial neural network model 900 may include random probability models, neural network models, and the like used in artificial intelligence learning methods such as machine learning and deep learning.

In one embodiment, the machine learning model for the welding defect inspection described above with reference to FIGS. 1 to 8 may be generated in the form of the artificial neural network model 900. For example, the artificial neural network model 900 may receive the optical measurement data measured using the optical device during welding of the target weldment and extract information associated with whether welding defects have occurred in the target weldment.

In one embodiment, the artificial neural network model 900 may be implemented as a multilayer perceptron (MLP) consisting of multiple layers of nodes and connections between them. The artificial neural network model 900 according to the present embodiments may be implemented using one of various artificial neural network model structures including, but not limited to, MLPs. As shown in FIG. 9, the artificial neural network model 900 includes an input layer 920 that receives input signals or data 910 from external sources, an output layer 940 that outputs output signals or data 950 corresponding to the input data, and hidden layers 930_1 to 930_n (where n is a positive integer) positioned between the input layer 920 and the output layer 940. The hidden layers 930_1 to 930_n receive the signals from the input layer 920, extract features, and pass them to the output layer 940. Then, the output layer 940 receives the signals from the hidden layers 930_1 to 930_n and outputs the signals to the external environment.

Learning methods for the artificial neural network model 900 may include a supervised learning method that is designed to train the artificial neural network model 900 to be optimized for solving a problem by using teaching inputs (e.g., labeled data), and an unsupervised learning method that is designed to train the artificial neural network model 900 without using teaching inputs (e.g., labeled data). In one embodiment, the artificial neural network model 900 may be trained based on a training data set that includes information associated with a plurality of welding conditions, reference optical measurement data measured using an optical device during welding of a reference weldment based on the plurality of welding conditions, and/or bead information of the reference weldment 330 where welding has been completed under each of the plurality of welding conditions.

In one embodiment, input variables of the artificial neural network model 900 may include optical measurement data. When such input variables described above are input through the input layer 920, output that is output from the output layer 940 of the artificial neural network model 900 may be information associated with the occurrence of welding defects.

As such, in the artificial neural network model 900, multiple input variables are respectively matched with corresponding multiple output variables in the input layer 920 and the output layer 940. By adjusting synaptic values between nodes included in the input layer 920, the hidden layers 930_1 to 930_n, and the output layer 940, the artificial neural network model 900 may be trained to extract a correct output corresponding to a particular input. Through this learning process, hidden features of the input variables of the artificial neural network model 900 can be identified and the synaptic values (or synaptic weights) between the nodes of the artificial neural network model 900 can be adjusted to reduce the error between the output variables calculated based on the input variables and the target output. Additionally, the artificial neural network model 900 may be trained with an algorithm that receives optical measurement data as input and may be further trained in a manner that minimizes loss between the output value and information (e.g., annotation information) associated with the occurrence of welding defects.

Using the trained artificial neural network model 900, information associated with the occurrence of welding defects can be extracted.

FIG. 10 is a flowchart illustrating a method 1000 for training a machine learning model (e.g., trained artificial neural network model 900) for welding defect inspection according to one embodiment of the present disclosure. The method 1000 may be performed by at least one processor.

The method 1000 may be initiated by the processor generating training data including reference optical measurement data measured using an optical device during welding of a reference weldment based on a plurality of welding conditions (step S1010). The optical device may include an infrared detection unit for detecting the heat of the weldment, a visible light detection unit for detecting the plasma of the weldment, and a spectroscopic unit for detecting wavelength-specific luminosity of the weldment (e.g., the intensity of light at different wavelengths of the weldment). The optical device may be configured as described herein in relation to FIGS. 1, 2, and 3.

Each of the plurality of welding conditions may be a condition that varies at least one of an output power of the welding laser, a focal point of the welding laser, or a spacing between weld targets of the weldment. At least some of the plurality of welding conditions may be normal welding conditions determined in advance (e.g., in advance of the welding).

The reference optical measurement data may include reference thermal data detected using the infrared detection unit, reference plasma data detected using the visible light detection unit, and reference wavelength-specific luminosity data detected using the spectroscopic unit, each of which are detected during welding of the reference weldment under each of the plurality of welding conditions.

The reference thermal data may include information associated with a contour extracted based on infrared intensity from an infrared image generated using the infrared detection unit during welding of the reference weldment. For example, the reference thermal data may include at least one of shape information, temperature information, or luminance information of the contour.

Additionally or alternatively, the reference thermal data may include information associated with a hot spot, which is a point of maximum infrared intensity in the infrared image generated using the infrared detection unit during welding of the reference weldment. The information associated with the hot spot may include at least one of brightness information of the hot spot region or positional information of the hot spot in the infrared image.

In one embodiment, the processor may obtain bead information of the reference weldment where welding has been completed under each of the plurality of welding conditions, and include the bead information in the training data. The bead information may be associated with the bead formed at the reference weldment. In this case, the bead information may include at least one of bead length information, bead depth information (e.g., depth information of the bead), or bead surface defect information (e.g., surface defect information of the bead).

Next, the processor may train a machine learning model for determining the occurrence of welding defects in the target weldment using the training data (step S1020).

The trained machine learning model may determine the occurrence of welding defects in and/or at the target weldment based on optical measurement data measured using an optical device during welding of the target weldment. The optical measurement data measured during welding of the target weldment may include thermal data of the target weldment detected using an infrared detection unit (e.g., detected heat), plasma data of the target weldment detected using a visible light detection unit, and wavelength-specific luminosity data of the target weldment detected using a spectroscopic unit. The thermal data, the plasma data, and the wavelength-specific luminosity data are detected during welding of the target weldment.

The trained machine learning model may determine the occurrence of a first defect type indicating defects associated with the welding conditions, the occurrence of a second defect type indicating defects associated with a length of a bead or a depth of the bead, and/or the occurrence of a third defect type indicating defects associated with surface defects of the bead.

The flowchart illustrated in and the above description in relation to FIG. 10 are merely an example, and implementations may vary in different embodiments. For example, the order of some steps may be changed, some steps may be omitted, other configurations may be added, and some steps described as being performed sequentially may be performed simultaneously.

According to some embodiments, a computing apparatus is provided which may include a non-transitory computer-readable storage medium or memory, and at least one processor connected to the storage medium or memory and configured to execute at least one computer-readable program included in the storage medium or memory. In some embodiments, the non-transitory computer-readable storage medium may store instructions for executing the method described in relation to FIG. 10 on a computer (e.g., of the computing apparatus).

According to some embodiments, elements that are described as performing an act may be configured to perform such an act.

Although the present disclosure has been described above with respect to embodiments thereof, the present disclosure is not limited thereto. Various modifications and variations can be made thereto by those skilled in the art within the present disclosure and the equivalent scope of the appended claims.

## Claims

1. A system comprising:
an optical device (130, 200, 340, 730) comprising:
an infrared detection unit (210) that detects heat generated at a weldment (120) during welding;
a visible light detection unit (230) that detects plasma generated at the weldment (120); and
a spectroscopic unit (250) that detects wavelength-specific luminosity at the weldment (120),
wherein the system is configured to determine an occurrence of welding defects (750) at the weldment (120) based on the detected heat, the detected plasma, and/or the detected wavelength-specific luminosity of the weldment (120).

2. The system as claimed in claim 1, wherein the system is configured that welding light (122, 332, 722) generated during welding of the weldment (120) passes through the infrared detection unit (210), the visible light detection unit (230), and the spectroscopic unit (250) in sequence.

3. The system as claimed in claim 1 or 2, wherein the infrared detection unit (210) comprises:
a first optical filter (212) that reflects or transmits a portion of welding light (122, 332, 722) generated during welding of the weldment (120); and
an infrared sensor (220) that receives a first portion of the welding light (122, 332, 722) reflected from the first optical filter (212),
wherein:
the visible light detection unit (230) is configured to receive a second portion of the welding light (122, 332, 722) that has transmitted through the first optical filter (212),
a wavelength band of the first portion is included in an infrared band, and
a wavelength band of the second portion is included in a visible light band.

4. The system as claimed in claim 3, wherein the wavelength band of the first portion is determined based on a type of welding target.

5. The system as claimed in any of the preceding claims, wherein the visible light detection unit (230) comprises:
a second optical filter (218) that splits received light; and
a visible light sensor (240) that receives a third portion of the welding light (122, 332, 722), the third portion being generated by splitting the second portion through the second optical filter (218),
wherein the spectroscopic unit (250) is configured to receive a fourth portion of the welding light (122, 332, 722), the fourth portion being generated by splitting the second portion through the second optical filter (218).

6. A method (1000) for training a machine learning model (140, 350, 700, 740, 800) for welding defect inspection, the method (1000) comprising:
generating training data including reference optical measurement data (342) measured using an optical device (130, 200, 340, 730) of any of the present claims during welding of a reference weldment (330) based on a plurality of welding conditions (310); and
training a machine learning model (140, 350, 700, 740, 800) for determining an occurrence of welding defects (750) in a target weldment (720) using the training data.

7. The method (1000) as claimed in claim 6, wherein each of the plurality of welding conditions (310) is a condition that varies at least one of an output power of a welding laser (112, 322, 712), a focal point of the welding laser (112, 322, 712), or a spacing between weld targets of the weldment (120).

8. The method (1000) as claimed in claim 6 or 7, wherein the reference optical measurement data (342) comprises:
reference thermal data detected using the infrared detection unit (210);
reference plasma data detected using the visible light detection unit (230); and
reference wavelength-specific luminosity data detected using the spectroscopic unit (250),
wherein the reference thermal data, the reference plasma data, and the reference wavelength-specific luminosity data are detected during welding of the reference weldment (330) under each of the plurality of welding conditions (310).

9. The method (1000) as claimed in claim 8, wherein the reference thermal data comprises information associated with a contour (512, 514, 516, 518) extracted based on infrared intensity from an infrared image generated using the infrared detection unit (210) during welding of the reference weldment (330).

10. The method (1000) as claimed in claim 6 or 9, wherein the reference thermal data comprises at least one of shape information, temperature information, or luminance information of the contour (512, 514, 516, 518).

11. The method (1000) as claimed in any of claims 8 to 10, wherein the reference thermal data comprises information associated with a hot spot (522, 532, 542),
wherein the hot spot (522, 532, 542) is a point of maximum infrared intensity in an infrared image generated using the infrared detection unit (210) during welding of the reference weldment (330), and
wherein the information associated with the hot spot (522, 532, 542) comprises at least one of brightness information of a region of the hot spot (522, 532, 542) or positional information of the hot spot (522, 532, 542) in the infrared image.

12. The method (1000) as claimed in any of claims 6 to 11, wherein the generating of the training data comprises:
obtaining bead information (334) associated with a bead (600) formed at a reference weldment (330) where welding has been completed under each of the plurality of welding conditions (310); and
including the bead information (334) in the training data, wherein the bead information (334) includes at least one of length (610) information of the bead (600), depth (620) information of the bead (600), or surface defect information of the bead (600).

13. The method (1000) as claimed in any of claims 6 to 12, wherein the machine learning model (140, 350, 700, 740, 800) determines the occurrence of welding defects (750) in the target weldment (720) based on optical measurement data (132, 732) measured using the optical device (130, 200, 340, 730) during welding of the target weldment (720).

14. The method (1000) as claimed in claim 13, wherein the optical measurement data (132, 732) measured during welding of the target weldment (720) comprises:
thermal data of the target weldment (720) detected using the infrared detection unit (210),
plasma data of the target weldment (720) detected using the visible light detection unit (230), and
wavelength-specific luminosity data of the target weldment (720) detected using the spectroscopic unit (250),
wherein the thermal data, the plasma data, and the wavelength-specific luminosity data are detected during welding of the target weldment (720).

15. The method (1000) as claimed in claim 13 or 14, wherein the machine learning model (140, 350, 700, 740, 800) determines a first occurrence of a first defect type indicating defects associated with the welding conditions (310), a second occurrence of a second defect type indicating defects associated with a length (610) of a bead (600) or a depth (620) of the bead (600), and/or a third occurrence of a third defect type indicating defects associated with surface defects of the bead (600).
